(19) Europäisches Patentamt
European Patent Office
Office européen des brevets

(11) **EP 3 557 223 A1**

(12) **EUROPEAN PATENT APPLICATION**

(43) Date of publication:
**23.10.2019 Bulletin 2019/43**

(51) Int Cl.:
*G01N 15/06* *(2006.01)*  *G01N 27/70* *(2006.01)*

(21) Application number: **19168955.3**

(22) Date of filing: **12.04.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **18.04.2018   JP 2018079882**

(71) Applicant: **NGK Spark Plug Co., Ltd.
Aichi 467-8525 (JP)**

(72) Inventors:
• **TANGE, Takeshi**
 **Nagoya-shi, Aichi 467-8525 (JP)**
• **GOTO, Yuichi**
 **Nagoya-shi, Aichi 467-8525 (JP)**

(74) Representative: **Diehl & Partner GbR
Patentanwälte
Erika-Mann-Strasse 9
80636 München (DE)**

(54) **PARTICULATE DETECTION APPARATUS AND PARTICULATE DETECTION METHOD**

(57) A particulate detection apparatus and method for detecting particulates contained in a gas under measurement, including a sensor section, a circuit section and a control section. The sensor section is configured to electrify the particulates contained in the gas under measurement with ions generated by corona discharge. The circuit section is configured to detect an ion current value which is a current value corresponding to an amount of ions having adhered to and having electrified the particulates discharged from the sensor section. The control section is configured to convert the ion current value to a particle-number concentration of the particulates and to convert the particle-number concentration to a mass concentration of the particulates using a correlation obtained in advance between particle number and mass of the particulates.

FIG. 6

EP 3 557 223 A1

**Description**

The present disclosure relates to a particulate detection apparatus and a particulate detection method.

A publicly known apparatus for detecting particulates such as PM (particulate matter) contained in exhaust gas discharged from an internal combustion engine of an automobile or the like employs a method of electrifying particulates in the exhaust gas using ions (see Patent Document 1).

In the above-mentioned particulate detection apparatus, ions generated in an ion generation section as a result of corona discharge are caused to adhere to particulates in the exhaust gas for electrification in an electrification chamber, and an ion current value corresponding to the amount of ions having adhered to and having electrified particulates discharged from the electrification chamber is detected. For detection of particulates, for example, the ion current value is converted to a particle-number concentration, and the particle-number concentration is further converted so as to determine the mass concentration of particulates.

Patent Document 1: Japanese Patent Application Laid-Open (*kokai*) No. 2015-108620

In a conventional particulate detection apparatus, the particle-number concentration is converted to a mass concentration with the center particle size (median diameter) of particulates set to a fixed value. Specifically, the particle-number concentration is converted to a mass concentration using the ratio between the particle-number concentration and the mass concentration (namely, correlation gain) obtained by analysis of the mass concentration and by analysis of the particle-number concentration at the center particle size.

Since the ion current value detected in the particulate detection apparatus is a numerical value based on the amount of electric charge at the surfaces of particles, the ion current value strongly correlates with the number of particles and depends on particle size. Therefore, the above-mentioned correlation gain changes with fluctuation of the center particle size.

Also, when the particle-number concentration increases, the ratio of particles having a large particle size increases, and the center particle size of particles becomes larger than the center particle size used for setting the correlation gain. Therefore, the discrepancy between the particle-number concentration and the mass concentration becomes larger, and consequently, the error in detecting the mass concentration of particulates becomes larger.

Meanwhile, the particle size distribution of particulates in exhaust gas changes depending on the type of a vehicle or the like. Therefore, in the case of a conventional particulate detection apparatus, the center particle size used for setting the correlation gain for calculating the mass concentration of particulates must be individually set, for example, for each vehicle model. Therefore, there is a need to improve the conventional particulate detection apparatus in terms of general versatility.

It is therefore an object of the present disclosure to provide a particulate detection apparatus and a particulate detection method which are highly versatile and which can accurately determine the mass concentration of particulates.

As a result of intensive study, the present inventors found that a certain correlation which cannot be represented by a simple ratio such as the above-described correlation gain exists between the particle number and mass of particulates contained in a gas under measurement.

The above object of the present disclosure has been achieved by providing (1) a particulate detection apparatus for detecting particulates contained in a gas under measurement. The particulate detection apparatus includes a sensor section, a circuit section, and a control section. The sensor section is configured to electrify particulates contained in the gas under measurement with ions generated by corona discharge. The circuit section is configured to detect an ion current value which is a current value corresponding to an amount of ions having adhered to and having electrified the particulates discharged from the sensor section. The control section is configured to convert the ion current value to a particle-number concentration of the particulates and to convert the particle-number concentration to a mass concentration of the particulates using a correlation obtained in advance between particle number and mass of the particulates.

By virtue of this configuration, use of the previously obtained correlation between the particle number and mass of the particulates allows accurate computation of the mass concentration from the particle-number concentration in consideration of the particle size dependency of the ion current value. Once the correlation is set, even when the particle size distribution of particulates in the gas under measurement changes, the accuracy in computing the mass concentration of the particulates can be maintained. Therefore, the general versatility of the particulate detection apparatus can be enhanced.

In a preferred embodiment (2) of the particulate detection apparatus (1), the correlation is represented by an approximation function in a predetermined form. By virtue of this configuration, the accuracy in computing the mass concentration of the particulates can be increased. Notably, an example of the approximation function in a predetermined form is a function using polynomial approximation, linear approximation, exponential approximation, power approximation, logarithmic approximation, or the like.

In another preferred embodiment (3) of the particulate detection apparatus (2) above, the approximation function is a function represented by the following equation (1):

$$Y = K1 \cdot X^2 + K2 \cdot X + K3 \qquad (1)$$

where X is the particle number of the particulates, Y is the mass of the particulates, and K1, K2, and K3 are constants. By virtue of this configuration, the accuracy in computing the mass concentration of the particulates can be increased easily without fail.

In yet another preferred embodiment (4) of the particulate detection apparatus of any of (1) to (3) above, the control section is configured to output the particle-number concentration or a particle number parameter based on the particle-number concentration, and the mass concentration or a mass parameter based on the mass concentration. This configuration allows accurate monitoring of both the particle number and mass of the particulates in the gas under measurement.

In a second aspect (5), the present disclosure provides a particulate detection method for detecting particulates contained in a gas under measurement. The particulate detection method comprises a step of electrifying the particulates contained in the gas under measurement with ions generated by corona discharge; a step of detecting an ion current value which is a current value corresponding to an amount of ions having adhered to and having electrified the particulates; a step of converting the ion current value to a particle-number concentration of the particulates; and a step of converting the particle-number concentration to a mass concentration of the particulates using a correlation obtained in advance between particle number and mass of the particulates.

By virtue of this configuration, by use of the correlation between the particle number and mass of the particulates, the accuracy in computing the mass concentration can be maintained even when the particle size distribution of particulates in the gas under measurement changes. Therefore, the mass concentration of the particulates can be computed accurately using a method common for various types of gases which undergo measurement.

The forgoing as well as other advantageous features of this disclosure will be more apparent from the following detailed description of exemplary embodiments with reference to the accompanying drawings, in which:

Figure 1 is a schematic view of a particulate detection apparatus according to an embodiment.

Figure 2 is a schematic sectional view of a sensor section of the particulate detection apparatus of Figure 1.

Figure 3 is a schematic diagram showing the circuit configuration of the particulate detection apparatus of Figure 1.

Figure 4 is an explanatory diagram showing an example of a conversion process performed by a control section of the particulate detection apparatus of Figure 1.

Figure 5 is a graph showing changes in the correlation between mass concentration and ion current value with particle size distribution.

Figure 6 is a graph showing the relation between the particle number and mass of particulates.

Figure 7 is a flowchart showing a particulate detection method according to the embodiment.

Reference numerals used to identify various features in the drawings include the following.

1: particulate detection apparatus, 2: sensor section, 3: drive section, 4: circuit section, 5: control section, 21: ion generation section, 21A: ion generation chamber, 21B: first electrode, 21C: air supply hole, 21D: nozzle, 22: electrification section, 22A: electrification chamber, 22B: inflow hole, 23: ion trapping section, 23A: trapping chamber, 23B: second electrode, 23C: discharge opening, 24: casing, 25: gas flow channel, 41: driver, 41A: discharge voltage control circuit, 41B: transformer drive circuit, 42: isolation transformer, 43: corona current measurement circuit, 44: ion current measurement circuit, 45A: first rectification circuit, 45B: second rectification circuit, 45C: third rectification circuit, 46: power supply, 47: signal line, 100: pipe.

Embodiments to which the present disclosure is applied will now be described with reference to the drawings. However, the present disclosure should not be construed as being limited thereto.

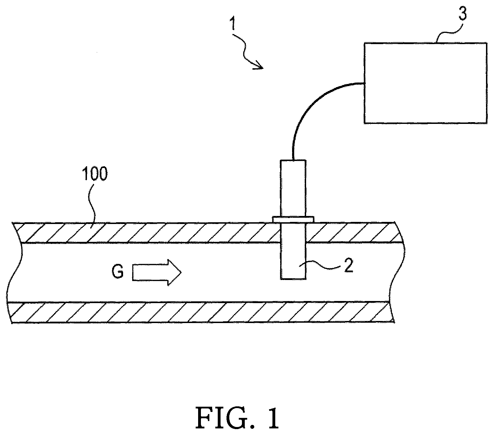

A particulate detection apparatus 1 shown in Figure 1 is an apparatus for detecting particulates contained in a gas under measurement G. The particulate detection apparatus 1 includes a sensor section 2 and a drive section 3.

No limitation is imposed on the gas under measurement for which the detection of particulates is performed by the particulate detection apparatus 1. However, an example of the gas under measurement is exhaust gas discharged from an internal combustion engine used in an automobile, an agricultural machine, a construction machine, a ship, a test station, a plant, or the like. The particulate detection apparatus 1 is preferably used for detection of PM (particulate matter), which are particulates contained in exhaust gas discharged from a gasoline engine or a diesel engine. Notably, the size of such particulates is, for example, about 0.1 μm to several tens of μm.

As shown in Figure 1, the sensor section 2 is inserted into a pipe 100 in which the gas under measurement G flows. The sensor section 2 is disposed in an exhaust gas flow passage to be located on the downstream side of a filter such as a DPF (diesel particulate filter).

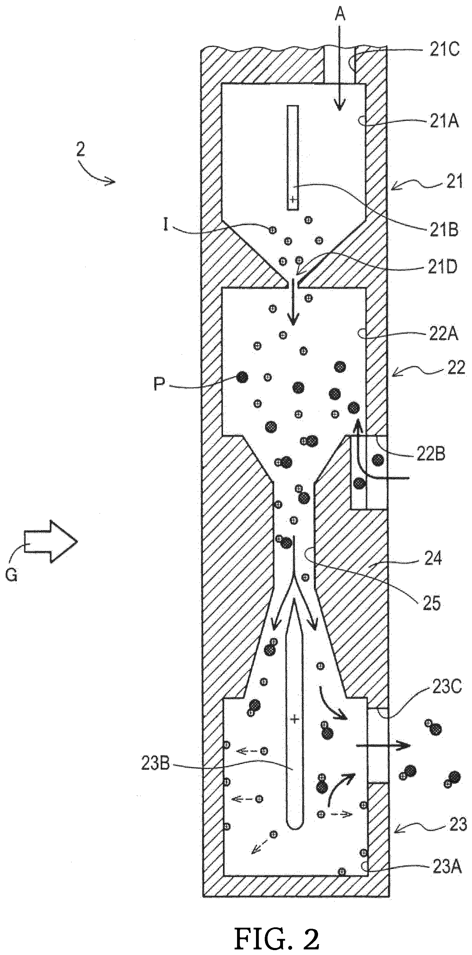

As shown in Figure 2, the sensor section 2 electrifies particulates P contained in the gas under measurement G with positive ions I produced by corona discharge. The sensor section 2 includes an ion generation section 21, an electrification section 22, and an ion trapping section 23.

The ion generation section 21, the electrification section 22, and the ion trapping section 23 are disposed in this order toward the forward end of the sensor section 2. An ion generation chamber 21A, the electrification section 22, and the ion trapping section 23 are provided in an electrically conductive casing 24.

The ion generation section 21 includes the ion generation chamber 21A and a rod-shaped first electrode 21B disposed in the ion generation chamber 21A. High-pressure air A is supplied to the ion generation chamber 21A through an air supply hole 21C. The high-pressure air A is supplied from a pump (not shown) provided separately from the sensor section 2.

In the ion generation section 21, corona discharge occurs as a result of application of DC voltage (for example, 2 to 3 kV) between the first electrode 21B serving as a positive electrode and the inner surface of the ion generation chamber 21A (namely, the inner wall of the casing 24) serving as a negative electrode. The corona discharge causes ionization of some molecules of the air A, whereby positive ions I are generated.

The positive ions I generated in the ion generation section 21 in this manner are supplied to the electrification section 22 through an orifice-shaped nozzle 21D provided at a central portion of the bottom surface of the ion generation chamber 21A.

The electrification section 22 has an electrification chamber 22A. The electrification chamber 22A communicates with the ion generation chamber 21A through the nozzle 21D. The gas under measurement G containing particulates P flows into the electrification chamber 22A through an inflow hole 22B.

Specifically, when the positive ions I and the air A flow into the electrification chamber 22A through the nozzle 21D, a negative pressure is created in the electrification chamber 22A. As a result, a certain amount of the gas under measurement G is drawn into the electrification chamber 22A through the inflow hole 22B. As a result, the gas under measurement G containing the particulates P and the air A containing the positive ions I are mixed.

As a result of the mixing, at least some of the particulates P are electrified by the positive ions I. The air containing the electrified particulates P and the positive ions I not used for the electrification is supplied to the ion trapping section 23 through a gas flow channel 25.

The ion trapping section 23 includes a trapping chamber 23A and a rod-shaped second electrode 23B disposed in the trapping chamber 23A. The trapping chamber 23A communicates with the interior of the pipe 100 through a discharge opening 23C.

The second electrode 23B, to which a voltage of about 100 V is applied, functions as an auxiliary electrode to assist in trapping of positive ions I not consumed for electrification of the particulates P. Specifically, as a result of application of the voltage between the second electrode 23B serving as a positive electrode and the inner surface of the trapping chamber 23A (namely, the inner wall of the casing 24) serving as a negative electrode, the positive ions I not consumed for electrification of the particulates P are subject to a repulsive force originating from the second electrode 23B and deviate to flow in a direction away from the second electrode 23B. The deviated positive ions I are trapped by the inner surface of the trapping chamber 23A.

Meanwhile, although the particulates P electrified by the positive ions I adhering thereto are also subject to the repulsive force originating from the second electrode 23B, due to their large mass as compared with the positive ions I, the electrified particulates P are not trapped in the trapping chamber 23A and are discharged to the outside of the sensor section 2 (namely, the interior of the pipe 100) through the discharge opening 23C as a result of the flow of the gas under measurement G.

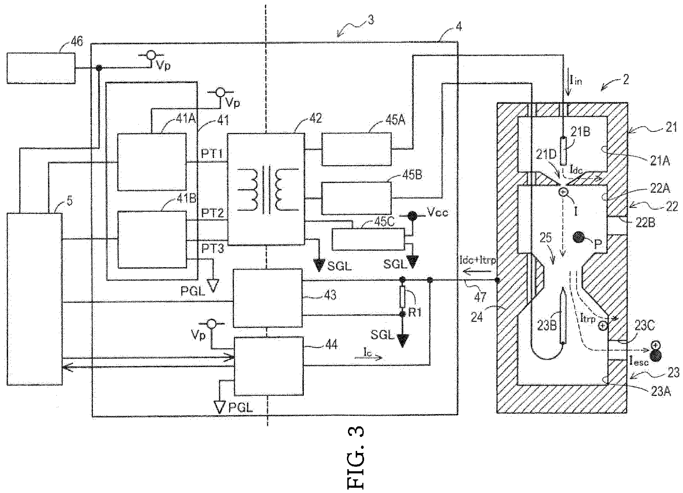

As shown in Figure 3, the drive section 3 includes a circuit section 4 for driving the sensor section 2 and a control section 5 for computing the particle-number concentration and mass concentration of the particulates P.

The circuit section 4 is configured to supply current for corona discharge to the sensor section 2 and to detect an ion current value which is a current value corresponding to the amount of ions having adhered to and having electrified the particulates P.

The circuit section 4 includes a driver 41, an isolation transformer 42, a corona current measurement circuit 43, an ion current measurement circuit 44, and three rectification circuits 45A, 45B, and 45C.

The isolation transformer 42 converts the AC voltage applied to its primary side to an AC voltage which is determined by the turn ratio between the primary and secondary coils of the isolation transformer 42 and is output from the secondary side of the isolation transformer 42. The primary and secondary coils of the isolation transformer 42 are insulated from each other.

As indicated by a broken line in Figure 3, the circuit section 4 is divided into a portion on the primary side of the solation transformer 42 and a portion on the secondary side of the solation transformer 42. The circuit on the primary side and the circuit on the secondary side are completely separate from each other and operate using independent power supplies.

The driver 41 is contained in the primary side circuit, and the control section 5 is connected to the primary side circuit. A power supply 46 supplies electric power to the primary side circuit. "Vp" in Figure 3 shows the connection to the power supply 46. The rectification circuits 45A, 45B, and 45C are contained in the secondary side circuit, and the sensor section 2 is connected to the secondary side circuit.

The corona current measurement circuit 43 and the ion current measurement circuit 44 extend between the circuit on the primary side of the isolation transformer 42 and the circuit on the secondary side of the isolation transformer 42 and are electrically connected to both the circuits.

Each of the primary side circuit and the secondary side circuit has a ground wiring, and the ground potentials (earth potentials) of the primary side circuit and the secondary side circuit are individually determined. The "inverted white triangles" in Figure 3 show the reference potential of the primary side circuit (hereinafter also referred to as the "primary side ground") PGL. The "inverted black triangles" in Figure 3 show the reference potential of the secondary side circuit (hereinafter also referred to as the "secondary side ground") SGL. An end of the secondary winding of the isolation transformer 42 is connected to the secondary side ground SGL.

The driver circuit 41 adjusts the electric power supplied to the primary side of the isolation transformer 42 and constitutes a push-pull-type power supply circuit in cooperation with the isolation transformer 42. The driver 41 includes a discharge voltage control circuit 41A and a transformer drive circuit 41B.

The discharge voltage control circuit 41A includes a DC/DC converter of an output voltage variable type (not shown), steps up the power supply voltage Vp output from the power supply 46, and applies the stepped-up voltage to the center tap PT1 of the primary winding of the isolation transformer 42. The output voltage of the DC/DC converter can be adjusted by the control section 5.

The transformer drive circuit 41B includes two switching elements connected to taps PT2 and PT3, respectively, which are provided at opposite ends of the primary winding of the isolation transformer 42. The two switching elements are disposed between the primary side ground PGL of the driver 41 and the two taps PT2 and PT3. The transformer drive circuit 41B converts the DC voltage supplied from the discharge voltage control circuit 41A to alternating current by alternatingly and repeatedly turning the two switching elements on and off.

Each of the first rectification circuit 45A and the second rectification circuit 45B rectifies the AC voltage output from the isolation transformer 42 for DC conversion. The DC voltage output from the first rectification circuit 45A is applied as a discharge voltage at the first electrode 21B. The DC current supplied from the first rectification circuit 45A is input current Iin flowing into the first electrode 21B.

The second rectification circuit 45B generates DC voltage through rectification and applies the DC voltage to the second electrode 23B. The voltage applied to the second electrode 23B changes when the voltage applied to the first electrode 21B is adjusted by the discharge voltage control circuit 41A.

The third rectification circuit 45C rectifies the AC voltage output from the isolation transformer 42 for DC conversion, thereby generating a drive voltage Vcc for operating an amplifier, etc., on the secondary side.

The corona current measurement circuit 43 detects the current value of discharge current (i. e., corona current) which flows due to the corona discharge generated in the ion generation section 21.

A shunt resistor R1 is connected to the secondary side of the corona current measurement circuit 43. A signal line 47 connected to the casing 24 of the sensor section 2 is connected to the secondary side ground SGL through the shunt resistor R1. The signal line 47 is also connected to the secondary side of the ion current measurement circuit 44.

Current Idc flowing from the first electrode 21B to the casing 24 and current Itrp corresponding to the electric charge of the positive ions I trapped by the casing 24 flow to the shunt resistor R1 through the signal line 47.

The ion current measurement circuit 44 supplies to the shunt resistor R1 ion current Ic which is equivalent to current Iesc corresponding to the positive ions I discharged from the sensor section 2 without being trapped in the ion trapping section 23. As a result, the input current Iin for discharge supplied from the first rectification circuit 45A to the first electrode 21B becomes equal to the total current collected from the sensor section 2. Thus, the current flowing out from the circuit section 4 and the current flowing into the circuit section 4 balance. Notably, the current Iesc is current which flows in accordance with the amount of ions having adhered to and having electrified the particulates P discharged from the sensor section 2.

The corona current measurement circuit 43 outputs the voltage of the shunt resistor R1 to the control section 5. The control section 5 feedback-controls the driver 41 based on the output of the corona current measurement circuit 43 such that the total current used for the corona discharge (i. e., the input current Iin) is maintained at a fixed value.

The ion current measurement circuit 44 outputs the ion current value (signal representing the detected ion current Ic) to the control section 5. The ion current measurement circuit 44 may output, as the ion current value, the current value of the ion current Ic as is or a voltage converted from the ion current Ic through use of, for example, an operational amplifier. In the case where an operational amplifier is used, a control signal is input to the ion current measurement circuit 44 from the control section 5.

The control section 5 includes a microprocessor and storage media such as RAM, ROM, etc. The control section 5 controls the particulate detection apparatus 1 by executing a program stored in advance.

The control section 5 controls the discharge voltage control circuit 41A and the transformer drive circuit 41B of the driver 41.

Also, the control section 5 detects the particulates P based on the ion current value, which is the output of the ion current measurement circuit 44.

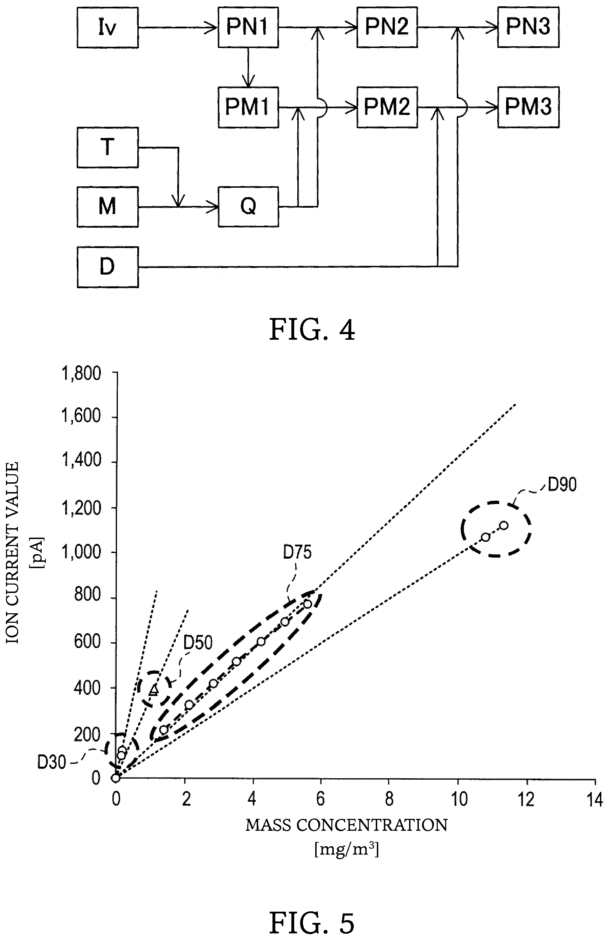

Specifically, as shown in Figure 4, the control section 5 first converts an ion current value Iv, which is a current value corresponding to the amount of ions having adhered to and having electrified the particulates P, to the particle-number concentration PN1 [count/cm$^3$] of the particulates P. The ion current value Iv converted by the control section 5 is the value output from the ion current measurement circuit 44 and a signal representing the magnitude of the ion current Ic.

The control section 5 converts the ion current value Iv to the particle-number concentration PN1 using a conversion equation obtained in advance. The conversion equation may be a conversion equation obtained from, for example, the number of ions which can adhere to and electrifies a single particulate P. However, the conversion equation is not limited thereto.

Next, the control section 5 converts the particle-number concentration PN1 [count/cm$^3$] to a mass concentration PM1 [mg/cm$^3$]. As shown in Figure 5, the correlation gain (the slope of a line representing the correlation between the ion current value (sensor output) and the mass concentration) changes depending on the distribution of particle size. Therefore, if the mass concentration is calculated using a simple (i. e., fixed) correlation gain, the accuracy is lowered. Notably, in Figure 5, "D30," "D50," "D75," and "D90" show the results of measurement for a particle size distribution having a peak particle size of 30 nm, a particle size distribution having a peak particle size of 50 nm, a particle size distribution having a peak particle size of 75 nm, and a particle size distribution having a peak particle size of 90 nm, respectively.

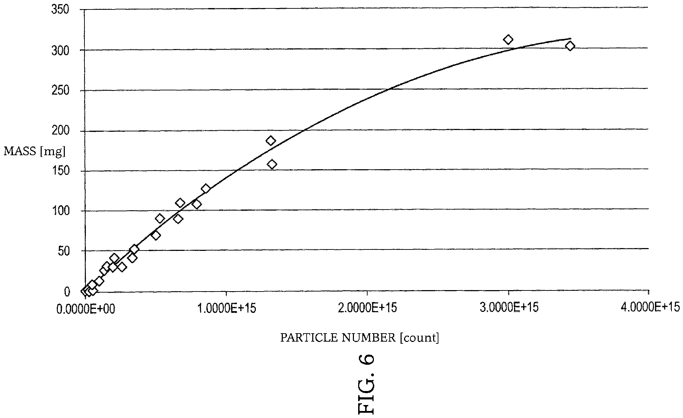

In view of the above, the control section 5 converts the particle-number concentration to the mass concentration of the particulates P using an approximation function in a predetermined form which represents a previously obtained correlation between the particle number and mass of the particulates P. For example, the approximation function is obtained through a field test. In the field test, each of different gases (for example, exhaust gases discharged from vehicles of different models) having different particle size distributions is supplied to a pipe as a gas under measurement so that the gas under measurement flows through the pipe. The number of particles (particulates) [count] and the mass [mg] of the particulates in the gas under measurement are measured using an analyzer, and the results of the measurement are plotted on a graph, from which an approximation curve is obtained as the approximation function. Figure 6 is an example of a graph showing the correlation between the particle number and mass of the particulates P.

When the particle number of the particulates is represented by X [count] and the mass of the particulates is represented by Y [mg], the approximation function representing the correlation between the particle number and mass of the particulates, which is obtained from the graph of Figure 6, is a quadratic polynomial which is represented by the following equation (1) using constants K1, K2, and K3.

$$Y = K1 \cdot X^2 + K2 \cdot X + K3 \qquad (1)$$

Notably, the approximation function representing the correlation between the particle number and mass of the particulates is not limited to the quadratic polynomial and may be a first order polynomial or a third or higher order polynomial. Also, the approximation function representing the above-described correlation may be any approximation function in a predetermined form such as a function using exponential approximation, power approximation, logarithmic approximation, or the like.

As shown in Figure 4, the control section 5 converts the particle-number concentration PN1 [count/cm$^3$] to a particle-number instantaneous value PN2 [count/s] which is the number of particles discharged per unit time using the flow rate of exhaust gas Q [m$^3$/s]. Furthermore, the control section 5 converts the particle-number instantaneous value PN2 to a particle-number emission PN3 [count/km] which is the number of particles in exhaust gas per unit travel distance of a vehicle using the travel distance D [km] of the vehicle. Notably, the exhaust gas flow rate Q is obtained from the output M [g/s] of a mass air flow sensor (MAF) through temperature correction based on the pipe temperature T [°C].

Similarly, the control section 5 converts the mass concentration PM1 [mg/cm$^3$] to mass instantaneous value PM2 [mg/s] which is the mass of particles discharged per unit time using the exhaust gas flow rate Q [m$^3$/s]. Furthermore, the control section 5 converts the mass instantaneous value PM2 to a mass emission PM3 [mg/km] which is the mass of particles in exhaust gas per unit travel distance of the vehicle using the travel distance D [km] of the vehicle. Notably, these conversions are not necessarily required.

The control section 5 is configured to output to the outside the particle-number concentration and/or particle number parameters based on the particle-number concentration (namely, the particle-number instantaneous value, the particle-number emission, etc.) and the mass concentration and/or mass parameters based on the mass concentration (namely, the mass instantaneous value, the mass emission, etc.).

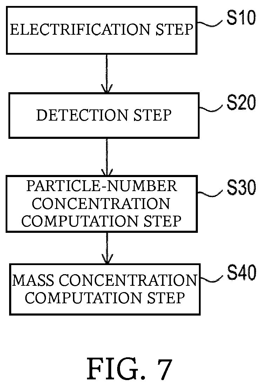

Figure 7 shows a particulate detection method of the present embodiment in which particulates contained in a gas under measurement such as exhaust gas discharged from a vehicle are detected using the particulate detection apparatus 1 shown in Figure 1. As shown in Figure 7, the particulate detection method of the present embodiment includes an electrification step S10, a detection step S20, a particle-number concentration computation step S30, and a mass concentration computation step S40.

In the electrification step S10, the particulates in the gas under measurement are electrified by ions generated as a result of corona discharge, in the above-described sensor section 2.

In the detection step S20, the ion current value which is the current value corresponding to the amount of ions having adhered to and having electrified the particulates is detected in the above-described circuit section 4.

In the particle-number concentration computation step S30, the ion current value is converted to the particle-number concentration of the particulates in the above-described control section 5.

In the mass concentration computation step S40, the particle-number concentration is converted to the mass concentration of the particulates in the above-described control section 5 using the previously obtained correlation between the particle number and mass of the particulates.

According to the embodiment having been described in detail above, the following effects are attained.

(1a) Use of the previously obtained correlation between the particle number and mass of the particulates allows accurate computation of the mass concentration from the particle-number concentration in consideration of the particle size dependency of the ion current value. Once the correlation is set, even when the particle size distribution of particulates in the gas under measurement changes, the accuracy in computing the mass concentration of the particulates can be maintained. Therefore, the general versatility of the particulate detection apparatus can be enhanced.

(1b) Since the particle-number concentration or particle number parameters based on the particle-number concentration and the mass concentration or mass parameters based on the mass concentration are output from the control section 5, both the particle number and mass of the particulates in the gas under measurement can be monitored accurately.

One embodiment of the present disclosure has been described; however, the present disclosure is not limited to the above-described embodiment, and various modifications are possible.

(2a) In the particulate detection apparatus 1 of the above-described embodiment, the means for representing the correlation between the particle number and mass of particulates used in the control section 5 is not limited to the approximation function. For example, a table which represents the correspondence between the particle number and mass of particulates may be used as the means for representing the correlation.

(2b) In the particulate detection apparatus 1 of the above-described embodiment, the control section 5 is not necessarily required to output the particle-number concentration or the particle number parameters based on the particle-number concentration. Namely, the particulate detection apparatus 1 may be configured to output only the mass concentration of particulates or the mass parameters based on the mass concentration.

(2c) The function of one constituent element in the above-described embodiment may be distributed to a plurality of constituent elements, or the functions of a plurality of constituent elements may be realized by one constituent element. Part of the configuration of the above-described embodiment may be omitted. Also, at least part of the configuration of the above-described embodiment may be added to or partially replace the configurations of other embodiments. Notably, all modes included in the technical idea specified by the wording of the claims are embodiments of the present disclosure.

Summarized, a particulate detection apparatus and method for detecting particulates contained in a gas under measurement are provided. The particulate detection apparatus comprises a sensor section, a circuit section and a control section. The sensor section is configured to electrify the particulates contained in the gas under measurement with ions generated by corona discharge. The circuit section is configured to detect an ion current value which is a current value corresponding to an amount of ions having adhered to and having electrified the particulates discharged from the sensor section. The control section is configured to convert the ion current value to a particle-number concentration of the particulates and to convert the particle-number concentration to a mass concentration of the particulates using a correlation obtained in advance between particle number and mass of the particulates.

The invention has been described in detail with reference to the above embodiments. However, the invention should not be construed as being limited thereto. It should further be apparent to those skilled in the art that various changes in form and detail of the invention as shown and described above may be made. It is intended that such changes be included within the spirit and scope of the claims appended hereto.

**Claims**

1. A particulate detection apparatus (1) for detecting particulates contained in a gas under measurement, comprising:

   a sensor section (2) configured to electrify particulates contained in the gas under measurement with ions generated by corona discharge;
   a circuit section (4) configured to detect an ion current value which is a current value corresponding to an amount of ions having adhered to and having electrified the particulates discharged from the sensor section; and
   a control section (5) configured to convert the ion current value to a particle-number concentration of the particulates and to convert the particle-number concentration to a mass concentration of the particulates using a correlation obtained in advance between particle number and mass of the particulates.

2. The particulate detection apparatus (1) as claimed in claim 1, wherein the correlation is represented by an approximation function in a predetermined form.

3. The particulate detection apparatus as (1) claimed in claim 2, wherein the approximation function is a function represented by the following equation (1):

$$Y = K1 \cdot X^2 + K2 \cdot X + K3 \qquad (1)$$

   where X is the particle number of the particulates, Y is the mass of the particulates, and K1, K2 and K3 are constants.

4. The particulate detection apparatus (1) as claimed in any one of claims 1 to 3, wherein the control section (5) is configured to output the particle-number concentration or a particle number parameter based on the particle-number concentration, and the mass concentration or a mass parameter based on the mass concentration.

5. A particulate detection method for detecting particulates contained in a gas under measurement, which comprises:

   electrifying the particulates contained in the gas under measurement with ions generated by corona discharge (S10);
   detecting an ion current value which is a current value corresponding to an amount of ions having adhered to and having electrified the particulates (S20);
   converting the ion current value to a particle-number concentration of the particulates (S30); and
   converting the particle-number concentration to a mass concentration of the particulates using a correlation obtained in advance between particle number and mass of the particulates (S40).

FIG. 1

FIG. 2

FIG. 3

FIG. 4

FIG. 5

FIG. 6

ELECTRIFICATION STEP — S10

DETECTION STEP — S20

PARTICLE-NUMBER CONCENTRATION COMPUTATION STEP — S30

MASS CONCENTRATION COMPUTATION STEP — S40

# FIG. 7

Europäisches
Patentamt

European
Patent Office

Office européen
des brevets

## EUROPEAN SEARCH REPORT

Application Number

EP 19 16 8955

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANTTI ROSTEDT: "Non-Collecting Electrical Sensor for Particle Concentration Measurement", AEROSOL AND AIR QUALITY RESEARCH, 1 January 2009 (2009-01-01), XP055223638, ISSN: 1680-8584, DOI: 10.4209/aaqr.2009.03.0023 * SENSOR DESIGN; page 471; figures 1, 2 * * page 475, column 2, line 10 - page 476, column 1, line 6; figure 9 * * page 475, column 1, line 1 - column 2, line 9 * | 1-5 | INV. G01N15/06 G01N27/70 |
| X | US 2015/120229 A1 (SUGIYAMA TAKESHI [JP] ET AL) 30 April 2015 (2015-04-30) * paragraphs [0118] - [0131]; figures 3-9 * | 1,5 | |
| X | JP 2016 075674 A (NGK SPARK PLUG CO) 12 May 2016 (2016-05-12) * paragraphs [0015] - [0041]; figures 1-3 * * paragraphs [0050] - [0066]; figures 5, 7-11 * | 1,5 | TECHNICAL FIELDS SEARCHED (IPC) G01N G01M |

The present search report has been drawn up for all claims

| Place of search | Date of completion of the search | Examiner |
|---|---|---|
| The Hague | 9 September 2019 | Bamière, François |

## ANNEX TO THE EUROPEAN SEARCH REPORT
## ON EUROPEAN PATENT APPLICATION NO.

EP 19 16 8955

This annex lists the patent family members relating to the patent documents cited in the above-mentioned European search report.
The members are as contained in the European Patent Office EDP file on
The European Patent Office is in no way liable for these particulars which are merely given for the purpose of information.

09-09-2019

| Patent document cited in search report | Publication date | Patent family member(s) | Publication date |
|---|---|---|---|
| US 2015120229 A1 | 30-04-2015 | DE 102014015634 A1<br>JP 6383248 B2<br>JP 2015108620 A<br>US 2015120229 A1 | 30-04-2015<br>29-08-2018<br>11-06-2015<br>30-04-2015 |
| JP 2016075674 A | 12-05-2016 | NONE | |

EPO FORM P0459

For more details about this annex : see Official Journal of the European Patent Office, No. 12/82

**EP 3 557 223 A1**

REFERENCES CITED IN THE DESCRIPTION

**Patent documents cited in the description**

- JP 2015108620 A